# EUROPEAN PATENT SPECIFICATION

(11) **EP 0 645 457 B1**
(45) Date of publication and mention of the grant of the patent: **01.04.1998**
(21) Application number: 94113948.7
(22) Date of filing: 06.09.1994
(51) Int. Cl.: C12P 13/24, C12N 9/02

(54) **L-proline-3-hydroxylase and process for producing cis-3-hydroxy-L-proline**
L-Proline-3-Hydroxylase und Verfahren zur Herstellung von cis-3-Hydroxy-L-Prolin
L-proline-3-hydroxylase et procédé de préparation de cis-hydroxy-3-L-proline

(30) Priority: 07.09.1993 JP 221941/93
(43) Date of publication of application: 29.03.1995
(73) Proprietor: KYOWA HAKKO KOGYO CO., LTD., Chiyoda-ku, Tokyo-to (JP)
(72) Inventor: Ozaki, Akio, Machida-shi, Tokyo (JP); Mori, Hideo, Machida-shi, Tokyo (JP); Shibasaki, Takeshi, Machida-shi, Tokyo (JP); Ando, Katsuhiko, Machida-shi, Tokyo (JP); Ochiai, Keiko, Ebina-shi, Kanagawa (JP); Chiba, Shigeru, Kawasaki-shi, Kanagawa (JP); Uosaki, Yoichi, Machida-shi, Tokyo (JP)
(74) Representative: Vossius, Volker, Dr.

(56) References cited:
- EP-A- 0 190 350
- EP-A- 0 555 475
- BIOCHEM BIOPHYS RES COMMUN 76 (2). 1977 275-281., TRYGGVASON K ET AL 'SEPARATION OF PROLYL- 3 HYDROXYLASE AND PROLYL-4 HYDROXYLASE ACTIVITIES AND THE 4 HYDROXY PROLINE REQUIREMENT FOR SYNTHESIS OF 3 HYDROXY PROLINE.'

### SEQUENCE LISTING

(1) GENERAL INFORMATION:
   (i) APPLICANT:
      (A) NAME: KYOWA HAKKO KOGYO CO., LTD.
      (B) STREET: 6-1, Ohtemachi 1-chome, Chiyoda-ku
      (C) CITY: Tokyo
      (E) COUNTRY: Japan
      (F) POSTAL CODE (ZIP): none
      (G) TELEPHONE: 0081-3-3201-7211
      (H) TELEFAX: 0081-3-3284-1968
      (I) TELEX: J24543HKKYOWA
   (ii) TITLE OF INVENTION: PROCESS FOR PRODUCING
      CIS-3-HYDROXY-L-PROLINE
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO: 1:

   (i) SEQUENCE CHARACTERISTICS:
      (A) LENGTH: 29 amino acids
      (B) TYPE: amino acid
      (D) TOPOLOGY: unknown
   (ii) MOLECULE TYPE: protein
   (v) FRAGMENT TYPE: N-terminal
   (xi) SEQUENCE DESCRIPTION: SEQ ID NO: 1:
   Met Cys Ser His Ile Leu Gly Arg Ile Glu Leu Asp Gln Glu Arg Leu
   1               5                   10                  15
   Gly Arg Asp Leu Glu Tyr Leu Ala Thr Val Pro Thr Val
               20                  25

## Description

### Background of the Invention

The present invention relates to a process for producing cis-3-hydroxy-L-proline. Cis-3-hydroxy-L-proline is useful as a starting compound for medicines and an additive to foods. The present invention also relates to a novel enzyme capable of catalyzing the hydroxylation of L-proline at the 3-position of L-proline. The novel enzyme is used in the above-mentioned process.

Heretofore, chemosynthetic methods of producing cis-3-hydroxy-L-proline are known [(J. Amer. Chem. Soc., 84, 3980 (1962); J. Amer. Chem. Soc., 85, 2824 (1963); Nature 289, 310 (1981); J. Org. Chem., 54, 1866 (1989); Acta Chemica Scandinavica, 43, 290 (1989)].

The conventional chemosynthetics methods for producing cis-3-hydroxy-L-proline are not satisfactory for industrial production, because of (1) the expensive raw materials, (2) too many the reaction steps, (3) the complicated procedures for isolating and purifying the product and/or (4) the lower productivity of cis-3-hydroxy-L-proline.

No chemosynthetic or biological method of producing cis-3-hydroxy-L-proline by hydroxylating L-proline both position-selectively and stereo-selectively, had been reported yet.

The object of the present invention is to provide an advantageous process for the production of cis-3-hydroxy-L-proline, and the second object of the present invention is to provide a novel enzyme which catalyzes the hydroxylation of L-proline at the 3-position and which is useful in the above process.

Tryggvason et al. [Biochem. Biophys. Res. Commun. 76, 275, (1977)] described the separation of the prolyl-3-hydroxylase activity and the prolyl-4-hydroxylase activity from each other in rat kidney cortex. The prolyl-3-hydroxylase catalyzed the formation of trans-3-hydroxyproline moieties in a consensus sequence within collagen.

### Summary of the Invention

The present invention provides a process for producing cis-3-hydroxy-L-proline, which comprises allowing L-proline to coexist with 2-ketoglutaric acid, a divalent iron ion and an enzyme source which catalyzes the hydroxylation of L-proline at the 3-position of L-proline in an aqueous medium to convert L-proline into cis-3-hydroxy-L-proline, and recovering the cis-3-hydroxy-L-proline from the aqueous medium. The present invention further provides a novel hydroxylase having the following physicochemical properties (hereinafter referred to as L-proline-3-hydroxylase):
(1) Action and Substrate Specificity:
   The enzyme catalyzes the hydroxylation of L-proline at the 3-position of L-proline in the presence of 2-ketoglutaric acid and a divalent iron ion to produce cis-3-hydroxy-L-proline.
(2) Optimum pH Range:
   The enzyme has an optimum pH range of 6.5 to 7.5, when it is allowed to stand at 30°C for 20 minutes.
(3) Stable pH Range:
   The enzyme is stable at pH values of 6.5 to 8.0, when it is allowed to stand at 4°C for 23 hours.
(4) Optimum Temperature Range:
   The optimum temperature is 35 to 40°C when it is allowed to stand at pH 7.0 for 15 minutes.
(5) Stable Temperature Range:
   The enzyme is inactivated, when it is allowed to stand at pH 7.5 and at 50°C for 30 minutes.
(6) Inhibitors:
   The enzyme is inhibited by metal ions of Zn⁺⁺, Cu⁺⁺, Co⁺⁺ and Ba⁺⁺ and ethylenediaminetetraacetic acid (EDTA).
(7) Activation:
   The enzyme does not need any cofactor for its activation. L-Ascorbic acid accelerates the activity of the enzyme.
(8) Km Value:
   Km value is 0.49 mM for L-proline and is 0.11 mM for 2-ketoglutaric acid, when determined in a 100 mM N-tris(hydroxymethyl)methyl-2-aminoethansulfonic acid (TES) buffer (pH 7.0) containing 5 mM L-ascorbic acid, 1 mM ferrous sulfate and a pre-determined amount of this enzyme.
(9) Isoelectric point:
   The enzyme has an isoelectric point of 4.3 by Phastsystem™.
(10) Molecular Weight:
   The enzyme has a molecular weight of 35,000 ± 5,000 daltons by sodium dodecylsulfate-polyacrylamide gel electrophoresis.
(11) N-terminal Amino Acid Sequence:
   The enzyme has an N-terminal amino acid sequence illustrated by Sequence Number 1.

### Detailed Description of the Invention

As the enzyme source to be used in the present invention, any microorganism can be used so long as it has an enzymatic activity of catalyzing the hydroxylation of L-proline at the 3-position of L-proline in the presence of 2-ketoglutaric acid and a divalent iron ion. As the microorganism having such activity, mention may be made of microorganisms belonging to the genus Streptomyces or Bacillus. The preferred strain of such microorganism includes Streptomyces canus ATCC 12647, Streptomyces canus ATCC 12646, Streptomyces sp. TH1 (FERM BP-4399), and Bacillus sp. TH2 and TH3 (FERM BP-4397 and FERM BP-4398, respectively). Specifically, a culture, cells or processed cells of these strains can be used. Further, a crude enzyme preparation from cells of the microorganism as mentioned above, a purified product of such enzyme preparation, an immobilized enzyme preparation, etc. can be used.

Streptomyces sp. TH1 (FERM BP-4399) was newly isolated by the present inventors from the soil. The microbiological properties of the strain TH1 is mentioned below.

### 1. Morphology:

The morphological properties when the strain was cultivated on various media at 28°C for 14 days are shown in Table 1 below.

**Table 1 -**

| Morphological Properties | | |
|---|---|---|
| 1) Hyphae | Formation of aerial hyphae | Yes |
| | Fragmentation and motility of aerial hyphae | Not observed |
| | Fragmentation and motility of substrate hyphae | Not observed |
| 2) Spores | Sporulation and Positions to which spores adhere | Adhered to aerial hyphae |
| | Formation of sporangia | Not observed |
| | Number of spores on its aerial mycelium | More than 10 |
| | Appearance of spore chain | spiral |
| | Characteristics of spores | |
| | Surface structure | Spiny |
| | Shape and size | |
| | ellipsoidal, 0.5 ∼ 0.7 × 1.0 ∼ 1.2 µm | |
| | Motility and fragella | Not observed |
| 3) Others | Chlamydospores | Not observed |
| | Synnemata | Not observed |
| | Pseudosporangia | Not observed |
| | Branching mode of hyphae | Simple branching |

### 2. Cultural Characteristics in Various Media:

The strain TH1 grows normally or vigorously on usual synthetic and natural media, while its substrate hyphae are pale pink (rose), orange or greenish brown. On some media, the strain often produces brown or blackish soluble pigments.

The cultural characteristics of the strain TH1, when the strain was cultivated on various media at 28°C for 14 days, are shown in Table 2. The designation of the colors has been made, according to the classification of colors indicated in Color Harmony Manual published by Container Corporation of America.

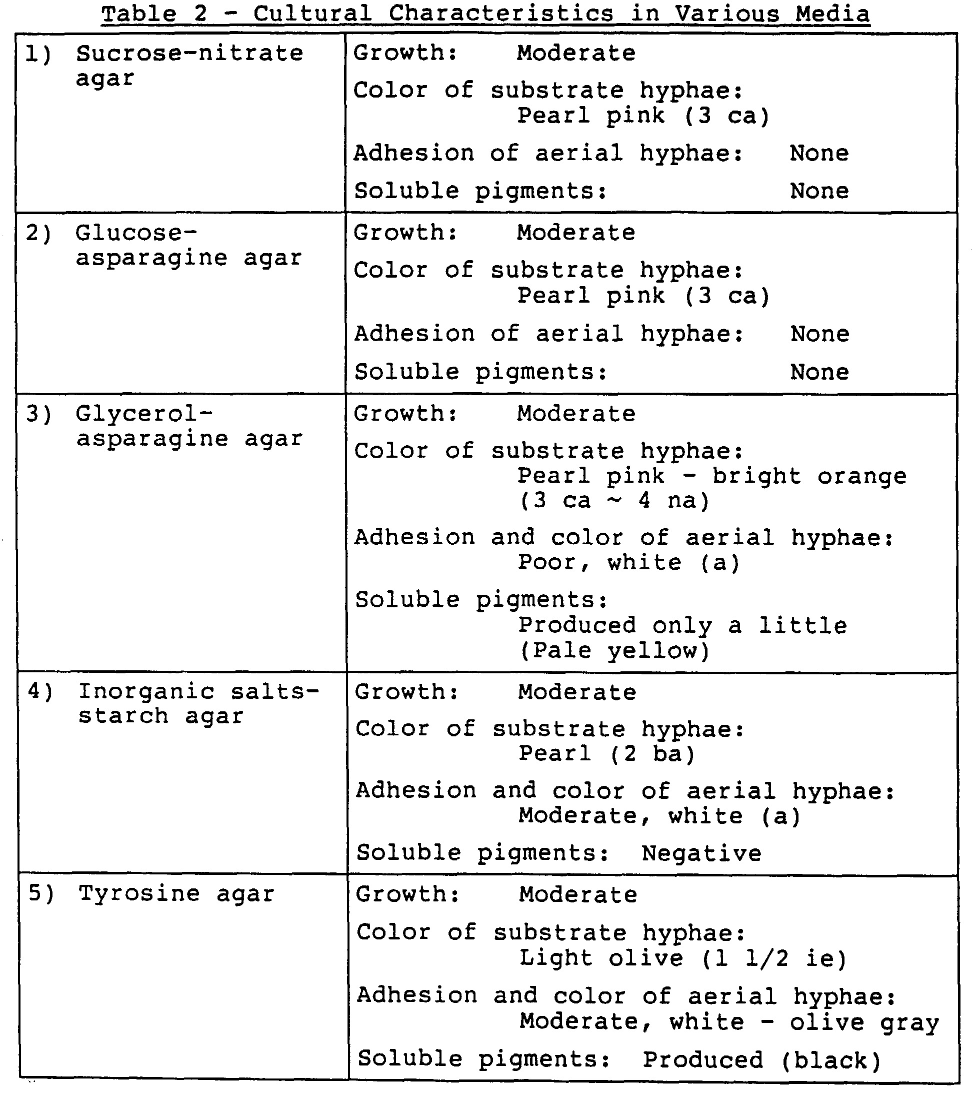

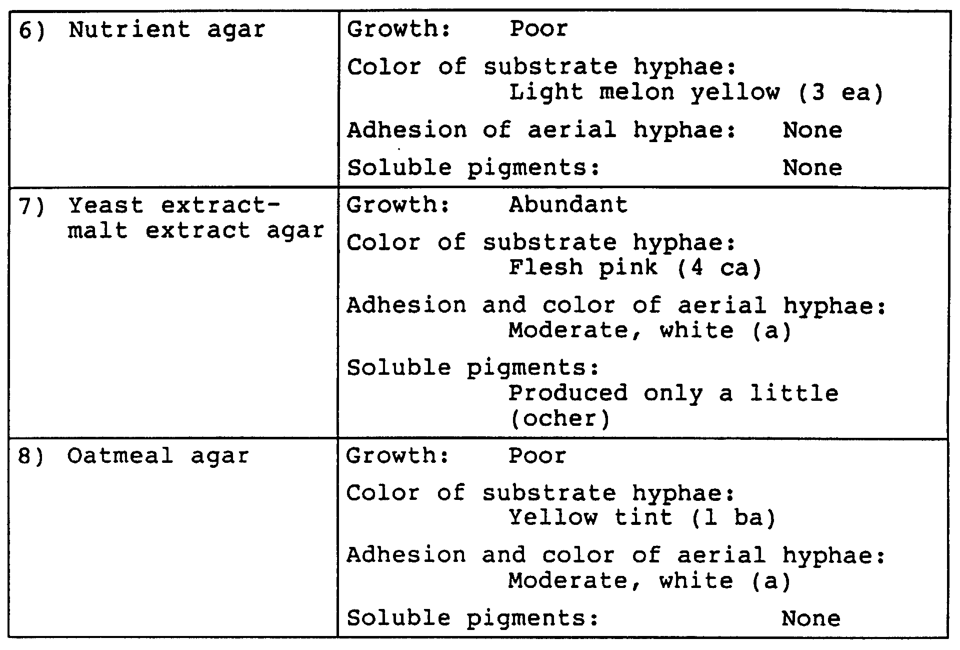

### 3. Physiological Properties:

The physiological properties of the strain TH1 is shown in Table 3, in which the "temperature range for growth" indicates the results of the strain after 7 day-cultivation by shaking. The remaining items indicate the results after 2 to 3 week-cultivation at 28°C.

**Table 3 -**

| Physiological Properties | | |
|---|---|---|
| 1) Temperature Range for Growth | | 23 ∼ 37°C |
| 2) Liquefaction of Gelatin | | No |
| 3) Hydrolysis of Starch | | Yes |
| 4) Coagulation of Skim Milk Powder | | No |
| 5) Peptonization of Skim Milk Powder | | No |
| 6) Formation of Melanoid Pigments | | |
| (1) Peptone-yeast extract-iron agar | | Yes |
| (2) Tyrosine agar | | Yes |
| 7) Utilization of Carbon Sources(*) | | |
| L-Arabinose | + | |
| D-Glucose | + | |
| D-Xylose | + | |
| Sucrose | + | |
| Raffinose | + | |
| D-Fructose | + | |
| Rhamnose | - | |
| Inositol : | + | |
| D-Mannitol | + | |

| | | |
|---|---|---|
| (*) The medium of Pridham Gottlieb was used as basal medium. + indicates that the strain utilized the carbon source; and - indicates that the strain did not utilize the carbon source. | | |

### 4. Chemotaxonomic Properties:

The configuration of diaminopimelic acid in the cells is LL type.

Accordingly, the strain TH1 is classified to the genus Streptomyces of actinomycetes in view of its morphological properties that it forms spiral spore chain consisting of more than ten spores on its aerial mycelium, and in view of its chemotaxonomic properties that the diaminopimelic acid contained in its cell wall is L,L-diaminopimelic acid (type I).

The strain TH1 was identified as Streptomyces sp. TH1, and the strain has been deposited with National Institute of Bioscience and Human-Technology, Agency of Industrial Science and Technology in Japan as of September 1, 1993 under FERM BP-4399 in terms of the Budapest Treaty.

Bacillus sp. TH2 (FERM BP-4397) and TH3 (FERM BP-4398) were newly isolated by the present inventors from the soil. The microbiological properties of the strains TH2 and TH3 are mentioned below.

Both the strains TH2 and TH3 have the following properties except that the cell sizes of the strain TH2 and TH3 are 1.5 to 1.8 × 3 to 4 pm and 1.2 to 1.5 × 3.5 to 4 pm, respectively, and the base compositions of DNA (G + C mol%) of the strains TH2 and TH3 are 36.46% and 37.74%, respectively.

### 1. Morphological Properties:

The strains had the morphological properties shown in Table 4 below.

**Table 4 -**

| Morphological Properties | | |
|---|---|---|
| 1) Cells | Morphology | Rod |
| | Polymorphism | Not observed |
| | Motility | Not observed |
| | (Position of flagella) | |
| 2) Endo Spores | Sporulation | Observed |
| | Morphology | Ellipse |
| | Positions | The end position |

### 2. Cultural Characteristics in Various Media:

The characteristics of the strains TH2 and TH3 are shown in Table 5 below.

**Table 5 -**

| Cultural Characteristics in Various Media | | |
|---|---|---|
| Bouillon-Agar Medium (Meat extract) | Growth | Abundant |
| | Shape of surface | Smooth |
| | Color | Pale yellow white |
| | Gloss | None |
| | Diffusible pigments | Negative |
| Bouillon-liquid Medium (Meat extract) | Growth appearance | Grown only a little on surface of the medium |
| | Turbidity | Positive |
| Bouillon-Gelatin-Medium | Liquefaction of gelatin | Positive |
| Litmus milk | Reaction | Acid |
| | Coagulation | Negative |
| | Liquefaction | Positive |

### 3. Physiological Properties:

The physiological properties of the strains TH2 and TH3 are shown in Table 6.

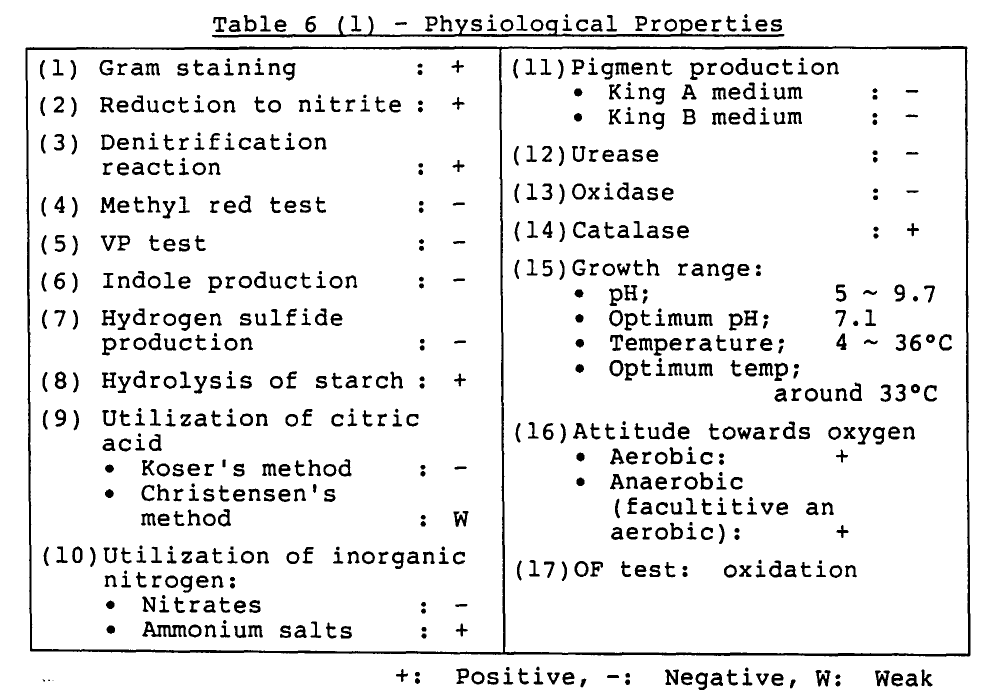

**Table 6 (2)**

| Production of Acid and Gas from Carbohydrates | | |
|---|---|---|
| | Acid production | Gas production |
| L-Arabinose | - | - |
| D-Xylose | - | - |
| D-Glucose | + | - |
| D-Mannose | - | - |
| D-Fructose | + | - |
| D-Galactose | - | - |
| Maltose | + | - |
| Sucrose | + | - |
| Lactose | - | - |
| Trehalose | + | - |
| D-Sorbitol | - | - |
| D-Mannitol | - | - |
| Inositol | - | - |
| Glycerol | - | - |
| Starch | + | - |
| +: Positive -: Negative | | |

### 4. The other Properties and the Chemotaxonomic Properties:

The other properties of the strains TH2 and TH3 are shown in Table 7, and the Chemotaxonomic properties are shown in Table 8 below.

**Table 7 -**

| Other Properties | |
|---|---|
| Degradation of Esculin | + |
| Degradation of Malonic acid | - |
| Degradation of Arginine | + |
| Decarboxylation of Lysine | - |
| Decarboxylation of Ornithine | - |
| Deamination of Phenylalanine | - |
| Resistance to NaCl (7.5%) | - |
| Phosphatase | + |
| +: Positive -: Negative | |

**Table 8 -**

| Chemotaxonomic Properties | | |
|---|---|---|
| 1) | Cellular lipids | |
| | • Ubiquinone | Negative |
| | • Menaquinone | MK-7 |
| | | |
| 2) | Diamino acid composition of cell wall peptidoglycan | meso-A₂ pm |

The strains having the microbiological properties mentioned above were compared with the strains indicated in Bergey's Manual of Systematic Bacteriology (Vol. 2, 1986).

Both the strains TH2 and TH3 are classified to the genus Bacillus of bacteria. The strains TH2 and TH3 were identified as Bacillus sp. TH2 and Bacillus sp. TH3, respectively. These strains have been deposited with the National Institute of Bioscience and Human-Technology, Agency of Industrial Science and Technology in Japan as of September 1, 1993 under FERM B-4397 for the strain TH2 and under FERM BP-4398 for the strain TH3, both in terms of the Budapest Treaty.

The medium for cultivating these microorganisms may be any of natural media and synthetic media, so long as it contains carbon sources, nitrogen sources, inorganic salts, etc. that may be assimilated by microorganisms having an activity of catalyzing the hydroxylation of L-proline to produce cis-3-hydroxy-L-proline.

As the carbon sources, carbohydrates such as glucose, fructose, sucrose, molasses containing these compounds, starch and starch hydrolysates, etc.; organic acids such as acetic acid, propionic acid, etc.; alcohols such as ethanol, propanol, etc. may be used.

As the nitrogen sources, ammonia; ammonium salts of various inorganic and organic acids such as ammonium chloride, ammonium sulfate, ammonium acetate, and ammonium phosphate, etc.; other nitrogen-containing compounds; peptone, meat extracts, yeast extracts, corn steep liquor, casein hydrolysates, soy bean cakes, soy bean cake hydrolysates, various cultured cells of microorganisms, their digested products, etc. may be used.

The inorganic material includes, for example, potassium dihydrogen phosphate, dipotassium hydrogen phosphate, magnesium phosphate, magnesium sulfate, sodium chloride, ferrous sulfate, manganese sulfate, copper sulfate, and calcium carbonate, etc.

The cultivation of these microorganisms is carried out under aerobic conditions, for example, by shaking culture or by submerged-aerial stirring culture. The temperature for the cultivation is preferably from 15 to 37°C, and the period for the cultivation is generally from 16 to 96 hours. During the cultivation, the pH of the medium is kept at 5.0 to 9.0 with inorganic or organic acids, alkaline solutions, urea, calcium carbonate, ammonia, etc.

The amount of the enzyme source to be used in the process for producing cis-3-hydroxy-L-proline depends on the amount of the substrate to be used in the process. Usually, it may be from 1.0 to 10,000,000 U, preferably from 1,000 to 2,000,000 U per one liter of the aqueous medium.

In the case of using microbial cells and products obtained by processing microbial cells, the concentration of wet cells to be used may be generally from 1 to 300 g/l.

The activity of the enzyme for producing one nmol of cis-3-hydroxy-L-proline for one minute under the conditions mentioned below is defined as one unit (U).

The enzyme preparation to be determined is added to 100 mM TES buffer (pH 7.0) containing 5 mM L-proline, 5 mM 2-ketoglutaric acid, 1 mM ferrous sulfate and 5 mM L-ascorbic acid to make 100 pl in total, and the mixture was allowed to stand at 35°C for 10 minutes. The reaction mixture is heated at 100°C for 2 minutes so as to stop the reaction, and the amount of cis-3-hydroxy-L-proline produced in the reaction mixture is determined by HPLC.

For the determination, any method capable of determining the amount of cis-3-hydroxy-L-proline may be employed. For instance, generally usable are a post-column derivatization method where HPLC is utilized, and a pre-column derivatization method where the compound to be determined in the reaction mixture is previously reacted with 7-chloro-4-nitrobenz-2-oxa-1,3-diazole chloride (hereinafter referred to as NBD) to form its NBD derivative, the derivative is separated by reversed-phase chromatography using HPLC and the thus-separated derivative is quantitatively determined by spectrofluorometry (excitation wavelength: 503 nm, emission wavelength: 541 nm). The pre-column derivatization method may be conducted, according to the method of William J. Lindblad & Robert F. Diegelmann, et al. (see Analytical Biochemistry, Vol. 138, pp. 390 - 395, 1984).

The concentration of L-proline to be used in the process for producing cis-3-hydroxy-L-proline may be from 1 mM to 2M.

The process needs a divalent iron ion. The concentration of the divalent iron ion may generally be from 1 to 100 mM. Any divalent iron ion may be used so long as it does not interfere the reaction. For instance, sulfides such as ferrous sulfate, chlorides such as ferrous chloride, ferrous carbonate, the salts of organic acids such as citrates, lactates, fumarates, etc. may be used.

The process also needs 2-ketoglutaric acid. 2-Ketoglutaric acid itself may be added to the reaction system, or alternatively, the compound that may be converted into 2-ketoglutaric acid by the metabolic activity of the microbial cells used in the reaction may be added thereto. The compound includes, for example, saccharides such as glucose, glutamic acid, succinic acid, etc. These compounds may be used singly or in combination.

The aqueous medium to be used in the process for producing cis-3-hydroxy-L-proline includes, for example, water, phosphates, carbonates, acetates, borates, citrates, buffers such as tris-buffers, alcohols such as methanol and ethanol, esters such as ethyl acetate, ketones such as acetone, amides such as acetamide, etc.

The reaction may be carried out in the culture medium where the above-mentioned microorganisms having an activity of catalyzing the hydroxylation of L-proline to produce cis-3-hydroxy-L-proline are being cultivated or have been cultivated, or alternatively, the reaction may also be carried out in an aqueous medium containing the cells of the above-mentioned microorganisms separated from the culture, processed cells, or a purified or crude enzyme derived from the cells.

Processed cells of the microorganisms include, for example, dried cells, lyophilized cells, surfactant-treated cells, enzymatically-treated cells, ultrasonically-treated cells, mechanically-ground cells, mechanically-compressed cells, solvent-treated cells, cellular protein fractions, immobilized cells, immobilized materials obtained by processing their cells, etc.

The enzyme reaction is generally carried out at a temperature of 15 to 50°C and at a pH of 6.0 to 9.0, for a period of 1 to 96 hours. If desired, surfactants and/or organic solvents may be added during the processing of the cells or during the reaction.

As the surfactants, mention may be made of cationic surfactants such as polyoxyethylene-stearylamine (e.g., Nymeen S-215, made by Nippon Oils and Fats Co.), cetyltrimethylammonium bromide, Cation FB, Cation F2-40E, etc.; anionic surfactants such as sodium oleylamidosulfate, Newrex TAB, Rapizole 80, etc.; ampholytic surfactants such as polyoxyethylene-sorbitan monostearate (e.g., Nonion ST221), etc.; other tertiary amines PB, hexadecyldimethylamine, etc. Any surfactant that may promote the reaction may be employed. The concentration of the surfactant to be employed in the reaction may be generally from 0.1 to 50 mg/ml, preferably from 1 to 20 mg/ml.

As the organic solvent, mention may be made of toluene, xylene, aliphatic alcohols, benzene, ethyl acetate, etc. Generally, the concentration of the organic solvent in the reaction system may be from 0.1 to 50 pl/ml, preferably from 1 to 20 pl/ml.

To recover cis-3-hydroxy-L-proline from the aqueous medium, ordinary separation methods such as column chromatography using ion-exchange resins, crystallization, etc. may be employed. The recovered cis-3-hydroxy-L-proline may be identified by ordinary analytic means using, for example, ¹³C-NMR spectrum ¹H-NMR spectrum, mass spectrum, specific rotatory power, etc.

Next, the novel enzyme, the L-proline-3-hydroxylase of the present invention is described below.

The L-proline-3-hydroxylase may be obtained by cultivating a microorganism having an ability to produce L-proline-3-hydroxylase in a medium so as to produce and accumulate the L-proline-3-hydroxylase in the culture medium, and recovering the L-proline-3-hydroxylase from the cells.

As the microorganism having an ability to produce L-proline-3-hydroxylase, mention may be made of a microorganism belonging to the genus Streptomyces or Bacillus and having such activity. Specific examples are Streptomyces canus ATCC12647, Streptomyces canus ATCC12646, Streptomyces sp. TH1, Bacillus sp. TH2 and Bacillus sp. TH3, a sub-cultivated strain thereof, the mutant thereof, its derivative thereof, etc.

The medium for cultivating these microorganisms may be any of natural media and synthetic media, so long as it contains carbon sources, nitrogen sources, inorganic salts, etc. that may be assimilated by the microorganism having an activity to produce L-proline-3-hydroxylase.

The carbon source includes, for example, carbohydrates such as glucose, fructose, sucrose, molasses containing these compounds, starch and starch hydrolysates, etc.; organic acids such as acetic acid, propionic acid, etc.; alcohols such as ethanol, propanol, etc. which may be assimilated by the microorganisms.

As the nitrogen source, ammonia; ammonium salts of various inorganic and organic acids such as ammonium chloride, ammonium sulfate, ammonium acetate, ammonium phosphate, etc.; other nitrogen-containing compounds; peptone, meat extracts, yeast extracts, corn steep liquor, casein hydrolysates, soy bean cakes, soy bean cake hydrolysates, various microorganisms for fermentation, their digested products, etc. may be used.

As the inorganic material, dipotassium hydrogen phosphate, potassium dihydrogen phosphate, magnesium phosphate, magnesium sulfate, sodium chloride, ferrous sulfate, manganese sulfate, copper sulfate, calcium carbonate, etc. can be used.

The cultivation of these microorganisms is carried out under aerobic conditions, for example, by shaking culture or by submerged-aeration stirring culture. The temperature of the cultivation is preferably from 15 to 37°C, and the period for the cultivation is generally from 16 to 96 hours.

During the cultivation, the pH of the medium is kept at 5.0 to 9.0. The pH of the medium is adjusted with inorganic or organic acids, alkaline solutions, urea, calcium carbonate, ammonia, etc. During the cultivation, L-proline may be added, if desired.

To isolate and purify the enzyme from the culture medium containing the enzyme, any ordinary method for isolating and purifying an enzyme may be employed. For instance, the culture is subjected to centrifugation to collect the cultivated cells therefrom, and the cells are fully washed and then disrupted by an ultrasonic cell disruptor, a French press, a Manton Gaulin homogenizer, a Dyno mill, etc. to obtain a cell-free extract. The cell-free extract is again subjected to centrifugation, and the resulting supernatant is then purified, for example, by salting-out with ammonium sulfate or the like, by anion-exchange chromatography with Diethylaminoethyl(DEAE)-Sepharose™ or the like, by hydrophobic chromatography with Butyl-Sepharose™, Phenyl-Sepharose™ or the like, by dye affinity chromatography with Red-Agarose or the like, by gel filtration with molecular sieves or by electrophoresis such as isoelectric point electrophoresis or the like. In this way, a pure product of the enzyme is obtained. The activity of the L-proline-3-hydroxylase thus isolated may be determined by the same method as mentioned above.

The L-proline-3-hydroxylase thus obtained, has the following physico-chemical properties (1) to (11):
(1) Action and Substrate Specificity:
   The enzyme catalyzes the hydroxylation of L-proline at the 3-position of L-proline in the presence of 2-ketoglutaric acid and a divalent iron ion to produce cis-3-hydroxy-L-proline.
(2) Optimum pH Range:
   The optimum pH range is 6.5 to 7.5 when it is allowed to stand at 30°C for 20 minutes.
(3) Stable pH Range:
   The enzyme is stable at pH values ranging from 6.5 to 8.0, when it is allowed to stand at 4°C for 23 hours.
(4) Optimum Temperature Range:
   The optimum temperature is in 35 to 40°C. when it is allowed to stand at pH 7.0 for 15 minutes.
(5) Stable Temperature Range:
   The enzyme is inactivated, when it is allowed to stand at pH 7.5 and at 50°C for 30 minutes.
(6) Inhibitors:
   The enzyme is inhibited by metal ions of Zn⁺⁺, Cu⁺⁺, Co⁺⁺ and Ba⁺⁺ and ethylenediaminetetraacetic acid (EDTA).
(7) Activation:
   The enzyme does not need any cofactor for its activation. L-Ascorbic acid accelerates the activity of the enzyme.
(8) Km Value:
   The Km value is 0.49 mM for L-proline and is 0.11 mM for 2-ketoglutaric acid, when determined in a 100 mM N-tris(hydroxymethyl)methyl-2-aminoethanesulfonic acid (TES) buffer (pH7.0) containing 5 mM L-ascorbic acid, 1 mM ferrous sulfate and a pre-determined amount of this enzyme.
(9) Isoelectric point:
   The enzyme has an isoelectric point of 4.3 by Phast™ system.
(10) Molecular Weight:
   The enzyme has a molecular weight of 35,000 + 5,000 daltons by sodium dodecylsulfate-polyacrylamide gel electrophoresis.
(11) N-terminal Amino Acid Sequence:
   The enzyme has an N-terminal amino acid sequence illustrated by Sequence Number 1.

The present invention is described in more detail by the following examples. However, the Examples are not intended to restrict the scope of the present invention.

### Example 1

### Production of Cis-3-hydroxy-L-proline:

SR3 medium comprising 1.0% glucose, 1.0% soluble starch, 0.5% yeast extract, 0.5% tryptone, 0.3% meat extract and 0.05% magnesium phosphate, and adjusted to pH 7.2 with 6N-NaOH, was put in test tubes (diameter 25 mm × length 200 mm) in an amount of 10 ml each and sterilized at 120°C for 20 minutes. One loopful of cells of Streptomyces sp. TH1, that had grown in HT-agar plate medium comprising 1% soluble starch, 0.2% NZ amine, 0.1% yeast extract, 0.1% meat extract and 1.5% agar, adjusted to pH 7.2 with 6N-NaOH, and sterilized at 120°C for 20 minutes, was inoculated into the SR3 medium in each test tube and cultivated at 28°C for 2 days by shaking culture. The resulting culture was used as a seed culture in the subsequent steps.

SR3 medium was put in 2 liter-Erlenmeyer flasks in an amount of 200 ml each and sterilized at 120°C for 20 minutes. The seed culture was inoculated in the SR3 medium in each Erlenmeyer flask and cultivated at 28°C for 2 days by shaking.

Df3 medium comprising 5% soluble starch, 3% corn steep liquor, 0.05% potassium dihydrogen phosphate, 0.05% magnesium sulfate 7-hydrate and 0.5% calcium carbonate, and adjusted to pH 7.0 with 6N-NaOH, was put in 5 liter-jar fermenters in an amount of 2 liters each and sterilized at 120°C for 20 minutes. The above-mentioned seed culture was inoculated in the DF3 medium in each jar fermenter under germ-free condition and cultivated under the condition of 700 rpm and 1 vvm, at 28°C for 2 days. During the cultivation, the pH of the medium was not adjusted. The thus-obtained culture medium was subjected to centrifugation at 15,000 × g for 10 minutes at 4°C and 75g of the wet cells thus separated were obtained per liter of the culture. The wet cells were washed with a physiological saline at 4°C and then again subjected to centrifugation and freezed at -80°C. The freezed cells were stored at the temperature until just before use.

One gram of the thus-obtained wet cells was suspended in 10 ml of a reaction mixture (a) [prepared by adding 1.4% (v/v) of Nymeen solution (prepared by dissolving 4g of Nymeen S-215 (made by Nippon Oils & Fats Co.) in 10 ml of xylene) to 100 mM TES buffer (pH 7.5) containing 5 mM L-proline, 5 mM 2-ketoglutaric acid, 5 mM L-ascorbic acid and 1 mM ferrous sulfate] and the mixture was allowed to stand at 30°C for 5 hours.

After completion of the reaction, the cells were removed from the reaction mixture by centrifugation, and the amount of hydroxyproline produced in the supernatant was determined.

The determination was carried out by HPLC under the conditions mentioned below. To identify the intended product, the resulting product was eluted from the column and reacted with NBD in the column line to form its NBD-derivative and the derivative was determined by spectrofluorometry.

### Conditions for determination by HPLC

[1] Apparatus Used:

| High Performance Liquid Chromatography Device (made by Shimadzu Seisakusho K.K.) | |
|---|---|
| Chromatopac | CR6A |
| System Controller | SCL-6B |
| Autoinjector | SIL-6B |
| Liquid Chromatograph Pump | LC-6A |
| Column Oven | CTO-6A |
| Chemical Reaction Box | CRB-6A |
| Spectrofluorometric Detector | RF-550A |

[2] Column Used:
SUMCHIRAL OA5000 (diameter 4.5 mm x length 250 mm, made by Sumika Chemical Analysis Service Limited)
[3] Conditions for Analysis:

| | | |
|---|---|---|
| 1) | Mobile Phase | aqueous solution of 1 mM copper sulfate |
| 2) | Flow Rate of Mobile Phase | 1.0 ml/min |
| 3) | Column Temperature | 38°C |
| 4) | Buffer | 0.3M boric acid buffer (pH 9.6) 25 mM ethylenediaminetetraacetic acid |
| 5) | Flow Rate of Buffer | 0.2 ml/min |
| 6) | NBD Chloride Solution | methanol solution of 0.5 g/liter |
| 7) | Flow Rate of NBD Chloride Solution | 0.5 ml/min |
| 8) | Reaction Temperature | 60°C |
| 9) | Reaction Time | about 3 min |
| 10) | Wavelength for Detection | |
| | excitation wavelength | 503 nm |
| | emission wavelength | 541 nm |
| 11) | Sample | 10 µl |

As a result of the determination, it was verified that 910 pM (119 mg/liter) of cis-3-hydroxy-L-proline was produced in the reaction mixture.

### Example 2

### Purification of Cis-3-hydroxy-L-proline:

100g of the wet cells which were obtained in Example 1 was suspended in 1 liter of the reaction mixture (a) as described in Example 1, that had been put in a 2 liter-beaker. The suspension was allowed to stand at 30°C for 5 hours with stirring.

After the reaction, the cells were removed from the reaction mixture by centrifugation, and hydroxyprolines produced in the supernatant were determined by the same method as described in Example 1. As a result of the determination, it was verified that 809 µM (106 mg/liter) of cis-3-hydroxy-L-proline was produced in the reaction mixture.

The supernatant was separated from the reaction mixture, adjusted to pH 4.5, and passed through a column packed with 200 ml of an ion-exchange resin, Diaion SK1B (NH₄⁺ type, made by Mitsubishi Kasei Corp.). The fractions containing cis-3-hydroxy-L-proline was concentrated under reduced pressure and then passed through a column packed with 20 ml of an ion-exchange resin, Diaion PA412 (OH-type, made by Mitsubishi Kasei Corp.). The fraction containing cis-3-hydroxy-L-proline was concentrated under reduced pressure, and adjusted to pH 9.6, and 10 vol.% of OPA solution (0.075g of o-phthalaldehyde (OPA)/ml ethanol solution) and 2 vol.% of β-mercaptoethanol solution (10% v/v aqueous solution) were added thereto. The mixture was allowed to stand at 60°C for 5 minutes, whereby impurities of primary amino acids contained therein were reated with OPA. The resulting mixture was passed through a column packed with 10 ml of Sepabeads SP207 (made by Mitsubishi Kasei Corp.), to separate cis-3-hydroxy-L-proline from the impurities of OPA-derivatized primary amino acids. The fraction containing cis-3-hydroxy-L-proline was concentrated under reduced pressure and again passed through a column packed with 20 ml of an ion-exchange resin, Diaion PA412 (OH-type, made by Mitsubishi Kasei Corp.) to separate a fraction containing cis-3-hydroxy-L-proline. The fraction was concentrated and dried to obtain 68 mg of cis-3-hydroxy-L-proline as white crystals (63%).

Physicochemical properties of the chemical compound mentioned above were given below.
- Specific rotation:: [α]_{D}²¹ = -93.4 (c=0.503, H₂O)
- FAB-mass spectrum:: 132 (M+H)⁺
- ¹³C-NMR spectrum (D₂O, 125 MHz) ppm:: 33.9, 44.5, 68.3, 71.6, 171.3
- ¹H-NMR spectrum (D₂O, 500 MHz) ppm:: 2.18 (1H), 2.27 (1H), 3.52 (1H), 3.62 (1H), 4.18 (1H), 4.77 (1H)

The above-mentioned molecular weight data of its mass spectrum and specific rotation, and the analytical results of its ¹³C-NMR spectrum and ¹H-NMR spectrum were found to be identical as the data as described in the prior art [see J. Biol. Chem., 241, 1300 (1966), J. Antibiotics, 45, 824 (1992)] and the data of cis-3-hydroxy-L-proline which was synthesized according to the methods of the prior art [see Liebigs Ann. Chem., 1881 (1979), Tetrahedron, 42, 2421 (1986)].

### Example 3

### Production of cis-3-hydroxy-L-proline:

L-Proline was hydroxylated by use of Streptomyces canus ATCC 12647, Streptomyces canus ATCC 12646, Bacillus sp. TH2 and Bacillus sp. TH3.

SR3 medium comprising 1.0% glucose, 1.0% soluble starch, 0.5% yeast extract, 0.5% tryptone, 0.3% meat extract and 0.05% magnesium phosphate, and adjusted to pH 7.2 with 6N-NaOH was put in test tubes (diameter 25 mm × length 200 mm) in an amount of 10 ml each and sterilized at 120°C for 20 minutes. One loopful of cells of each of the microorganisms mentioned above that had grown in HT-agar plate medium was inoculated in the medium in a test tube and cultivated at 28°C for 2 days by shaking. The resulting culture was used as a seed culture in the following steps.

Separately, Df4 medium comprising 2.5% glycerol, 2.5% glucose, 1.5% soybean meal, 0.05% dipotassium hydrogen phosphate, 0.05% magnesium sulfate 7-hydrate and 0.5% calcium carbonate, and adjusted to pH 7.0 with 6N-NaOH, was put in test tubes (diameter 25 mm × length 200 mm) in an amount of 10 ml each and sterilized at 120°C for 20 minutes. One ml of the seed culture was inoculated in the medium in each test tube under germ-free condition and cultivated at 28°C for 1 day by shaking. The thus-obtained culture was subjected to centrifugation at 15,000 × g for 10 minutes at 4°C. The cells thus separated were washed with 80 mM TES buffer (pH 7.5) and then again subjected to centrifugation. The thus-obtained wet cells were suspended in 1 ml of the reaction mixture (a) and allowed to stand at 30°C for 3 hours.

As a result, it was verified that 242 µM, 202 µM, 318 µM and 141 µM of cis-3-hydroxy-L-proline were produced in the reaction mixture by use of the strains ATCC12647, ATCC12646, TH2 and TH3 respectively.

### Example 4

### Production of Cis-3-hydroxy-L-proline:

In the same manner as in Example 3 except that Df2 medium comprising 5% soluble starch, 1.5% dry yeast, 0.05% potassium dihydrogen phosphate, 0.05% magnesium sulfate 7-hydrate and 0.5% calcium carbonate and adjusted to pH 7.0 with 6N-NaOH, and further containing 0.1% L-proline was used in place of Df4 medium, each of Bacillus sp. TH2 and TH3 were cultivated.

As a result, it was verified that 745 µM (97.6 mg/l) of cis-3-hydroxy-L-proline for the strain TH2 and 327 µM (42.8 mg/l) for strain TH3 were produced in the aqueous medium.

### Example 5

### Isolation and Purification of L-proline-3-hydroxylase:

### (1) Preparation of Cell-free Extract:

Thirty grams of the freezed cells obtained in Example 2 was thawed out and suspended in 200 ml of Buffer (A) [20 mM piperazine buffer adjusted to 5.3 with 6N-HCl containing 1 mM dithiothreitol (DTT), 0.2 mM EDTA, 0.1% (v/v) Tween 20 and 10% (v/v) glycerol] while cooling with ice. The cells in the resulting suspension was disrupted by means of an ultrasonic cell disruptor while cooling with ice. The thus-disrupted cell suspension was subjected to centrifugation at 30,000 × g at 4°C for 30 minutes to separate a supernatant.

The subsequent operations were conducted under cooling with ice or at a temperature of 4°C or lower.

### (2) Isolation and Purification by Column Chromatography:

(2)-1 Acid Treatment
The supernatant obtained in the previous step was adjusted to pH 4.5 with 6N-HCl, and centrifuged at 15,000 × g for 30 minutes to obtain the precipitates formed by the above treatment.
(2)-2 Resource Q™ Column Chromatography (I):
The supernatant obtained in the previous step was passed through a RESOURCE™ Q column (6 ml; made by Pharmacia Co.) that had been equilibrated with Buffer (A). The column was washed with Buffer (A), and the fraction containing the enzyme was eluted with Buffer (A) having a linear concentration gradient of NaCl of 0 to 0.3M.
(2)-3 Resource Q™ Column Chromatography (II):
The active fraction obtained in the previous step was diluted three times with Buffer (B) [20 mM TES buffer (pH 7.5) containing 1 mM DTT, 0.2 mM EDTA and 10% (v/v) of glycerol] and was passed through a RESOURCE™ Q column (1 ml; made by Pharmacia Co.) that had been equilibrated with Buffer (B). The column was washed with Buffer (B), and the fraction containing the enzyme was eluted with Buffer (B) having a linear concentration gradient of NaCl of 0 to 0.3M.
(2)-4 Phenyl Sepharose™ Column Chromatography:
NaCl was added to the active fraction obtained in the previous step so that the resulting solution had an NaCl concentration to 2M. The mixture was applied to a Phenyl Sepharose™ column (1 ml; Phenyl Sepharose HP™ HiLoad) that had been equilibrated with Buffer (B) containing 2M NaCl. The column was washed with Buffer (B) containing 2M NaCl, and the fraction containing the enzyme was eluted with Buffer (B) containing 0.1% (v/v) of Tween® 20.
(2)-5 Resource Q Column Chromatograph (III):
The active fraction obtained in the previous step was de-salted using a PD-10 column (1 ml; made by Pharmacia Co.), and the resulting mixture was passed through a RESOURCE™ Q column (1 ml) that had been equilibrated with Buffer (A). After the column was washed with Buffer (A), the fraction containing the enzyme was eluted with Buffer (A) having a linear concentration gradient of NaCl of 0 to 0.3M.
(2)-6 Resource Q Column Chromatography:
The active fraction obtained in the previous step was diluted three times with Buffer (B) containing 0.1% (v/v) Tween 20 and passed through a RESOURCE™ Q column (1 ml; made by Pharmacia Co.) that had been equilibrated with Buffer (B). The fraction containing the enzyme was eluted with Buffer B having a linear concentration gradient of NaCl of 0 to 0.3M.
The foregoing steps for the isolation and purification of the L-proline-3-hydroxylase are summarized in Table 9.

**Table 9**

| Isolation and Purification of L-Proline-3-Hydroxylase | | | | |
|---|---|---|---|---|
| Fraction | Total Protein (mg) | Total Activity (U) | Specific Activity (U/mg of protein) | Yield (%) |
| Cell-free Extract | 542 | 3540 | 6.5 | 100 |
| | | | | |
| pH 4.5 treated supernatant | 106 | 1800 | 17 | 56 |
| | | | | |
| Resource Q (I) pH 5.3 | 7.5 | 1553 | 207 | 44 |
| | | | | |
| Resource Q (II) pH 7.5 | 3.3 | 1036 | 306 | 29 |
| | | | | |
| Phenyl Sepharose | 0.43 | 188 | 437 | 5.3 |
| | | | | |
| Resource Q (III) pH 5.3 | 0.16 | 90.5 | 566 | 2.6 |
| | | | | |
| Resource Q (IV) pH 7.5 | 0.035 | 60.3 | 1723 | 1.7 |

### Example 6

### Properties of L-Proline-3-Hydroxylase:

(1) Analysis by Electrophoresis:
   The purified enzyme preparation obtained in Example 5 was analyzed by sodium dodecylsulfate-polyacrylamide gel electrophoresis, using polyacrylamide gel PAGEL NPU-12.5L made by Atto Co. and SDS-PAGE Molecular Weight Standard, Broad Range made by Biorad Co. As a result, it was verified that the enzyme was composed of almost uniform sub-unit having a molecular weight of about 35,000 ± 5,000 daltons.
(2) Properties of the Enzyme:
   Using the reaction mixture mentioned below, the enzyme was subjected to the substrate omission and addition tests, by which the compounds indispensable to the enzyme reaction for hydroxylating L-proline at the 3-position of L-proline, the promoters for the enzyme reaction and the inhibitors against the enzyme reaction were investigated.

The reaction mixture was composed of 100 mM TES buffer (pH 7.0) containing 5 mM L-proline, 5 mM 2-ketoglutaric acid, 1 mM ferrous sulfate, 5 mM L-ascorbic acid and a pre-determined amount of the pure enzyme, the total volume being 100 pl. The reaction was initiated by addition of the enzyme and continued for 10 minutes at 35°C. The reaction was stopped by heating the reaction mixture at 100°C for 2 minutes. The amount of cis-3-hydroxy-L-proline formed in the reaction mixture was determined by the pre-column derivatization method using HPLC. One hundred microliters of 0.3M boric acid buffer (pH 10.7), 4 pl of an aqueous solution of 10% (v/v) mercaptoethanol and 16 p1 of ethanol solution of 5% (w/v) OPA were added to 100 pl of the reaction mixture, and the mixture was allowed to stand at 60°C for 30 seconds. In addition, 50 pl of ethanol solution of 2% (w/v) NBD chloride were added thereto and the mixture was allowed to stand at 60°C for 40 minutes. The reaction was stopped by adding 30 µl of 1 N-hydrochloric acid to the reaction mixture, and the precipitates formed were removed therefrom by centrifugation and filtration. The cis-3-hydroxy-L-proline formed by the reaction was quantitatively determined by HPLC analysis.

The HPLC was carried out for the determination under the following conditions:

| | |
|---|---|
| Mobile Phase | 10 mM Citric Acid (pH 4.0)/Methanol = 3/1 (v/v) |
| Flow Rate | 1 ml/min |
| Column | YMC Pack ODS AQ-312 (made by YMC Co., 6 × 150 mm) |
| Column Temperature | 50°C |
| Detection | Spectrofluorometry (excitation wavelength: 503 nm, emission wavelength: 541 nm) |

The test results verified that L-proline, 2-ketoglutaric acid and Fe⁺⁺ ion are indispensable for the enzyme reaction for hydroxylating L-proline at the 3-position of L-proline, that L-ascorbic acid promotes the enzyme reaction and that Zn⁺⁺, Cu⁺⁺, Co⁺⁺, Ba⁺⁺ and EDTA inhibit the reaction.

The test results are shown in Table 10.

**Table 10**

| Components Influencing Enzyme Reaction of L-Proline-3-Hydroxylase | | |
|---|---|---|
| Components in Reaction Mixture | Added (+)²⁾ Not Added (-) | Relative Activity³⁾ |
| Basic Reaction Mixture¹⁾ | | 100 |
| | - Pure Enzyme | 0 |
| | - L-proline | 0 |
| | - 2-Ketoglutaric Acid | 0 |
| | - Fe⁺⁺ | 0 |
| | - L-Ascorbic Acid | 25 |
| | + 2 mM EDTA | 5 |
| | + 1 mM Zn⁺⁺ | 0 |
| | + 1 mM Cu⁺⁺ | 4 |
| | + 1 mM Co⁺⁺ | 13 |
| | + 1 mM Ba⁺⁺ | 42 |

| | | |
|---|---|---|
| 1) The standard reaction mixture was composed of 100 mM TES buffer (pH 7.0) containing 5 mM L-proline, 5 mM 2-ketoglutaric acid, 1 mM ferrous sulfate, 5 mM L-ascorbic acid and a pre-determined amount of the pure enzyme, the total volume being 100 µl. The reaction was carried out at 35°C for 10 minutes. | | |
| 2) "(+)" means that the reaction mixture contained the component shown in the table. "(-)" means that the reaction mixture did not contain the component shown in the table. The concentration shown in the table means the concentration of the component in the reaction mixture. | | |
| 3) The activity is indicated as the relative activity, the activity in the standard reaction mixture being defined as 100. | | |

### (3) Optimum pH Range:

In the determination of the enzymatic activity of the L-proline-3-hydroxylase, the reaction was carried out while the buffer component in the reaction mixture was changed to MES buffer [2-(N-morpholino)ethanesulfonic acid] at pH of 5.5 to 6.5, it was changed to PIPES buffer [piperazine-N,N'-bis(2-ethanesulfonic acid] at pH of 6.5 to 7.5, it was changed to TES buffer at pH of 7.0 to 8.0, and it was changed to TAPS buffer [N-tris(hydroxymethyl)methyl-3-aminopropanesulfonic acid] at pH of 8.0 to 9.0. As a result, the enzyme had an activity of more than 80% of the maximum activity thereof at pH ranging from pH 6.5 to 7.5. The detailed test results are shown in Table 11 below.

**Table 11**

| Optimum pH Range for the Enzyme Reaction | | |
|---|---|---|
| pH | (buffer) | Relative Activity¹⁾ |
| 5.5 | (MES) | 15.0 |
| 6.0 | (MES) | 70.9 |
| 6.5 | (PIPES) | 83.3 |
| 7.0 | (PIPES) | 92.1 |
| 7.0 | (TES) | 100 |
| 7.5 | (PIPES) | 80.4 |
| 7.5 | (TES) | 85.0 |
| 8.0 | (TES) | 72.2 |
| 8.0 | (TAPS) | 76.3 |
| 8.5 | (TAPS) | 52.9 |
| 9.0 | (TAPS) | 44.5 |

| | | |
|---|---|---|
| 1) The activity is indicated as a relative activity, defining the maximum activity as 100. | | |

### (4) Stable pH Range:

The enzyme solution in Buffer (B) was diluted three times with 100 mM of a buffer (MES buffer at pH of 5.5 to 6.5, PIPES buffer at pH of 6.1 to 7.5, TES buffer at pH of 7.0 to 8.0, TAPS buffer at pH of 8.0 to 9.0), kept at 4°C for 23 hours, and then its activity was measured. The enzyme kept at pH ranging from 6.5 to 8.0 had an activity of 80% or more of the original activity of the enzyme before the test. Accordingly, the enzyme was kept stable at pH ranging from 6.5 to 8.0.

### (5) Optimum Temperature Range:

In the determination of the enzyme activity of the L-proline-3-hydroxylase, the reaction was carried out at a temperature ranging from 15 to 50°C for 15 minutes. As a result, the enzyme had an activity of 80% or more of the maximum activity thereof at temperatures ranging from 35 to 40°C. The detailed test results are shown in Table 12 below.

**Table 12**

| Temperature Range for the Enzyme Reaction | |
|---|---|
| Reaction Temperature (°C) | Relative Activity¹⁾ |
| 15 | 19 |
| 20 | 29 |
| 25 | 53 |
| 30 | 74 |
| 35 | 100 |
| 40 | 89 |
| 45 | 64 |
| 50 | 28 |

| | |
|---|---|
| 1) The activity is indicated as a relative activity, the maximum activity being defined as 100. | |

### (6) Stable Temperature Range:

The enzyme was kept in Buffer (B) at temperatures ranging from 0 to 60°C for 30 minutes and thereafter the activity of the enzyme was determined. As a result, the enzyme was inactivated after it was kept at 50°C or higher for 30 minutes.

### (7) Km Value:

The Km value is 0.49 mM for L-proline and is 0.11 mM for 2-ketoglutaric acid, when determined in a TES buffer (pH 7.0) containing 5 mM L-ascorbic acid, 1 mM ferrous sulfate and a pre-determined amount of this enzyme.

### (8) Isoelectric point:

The enzyme was analyzed, using Phast™ system (made by Pharmacia Co.), to determine the isoelectric point of the enzyme. As a result, the isoelectric point of the enzyme was 4.3.

### (9) N-terminal Amino Acid Sequence:

The enzyme was analyzed, using Protein Sequencer Model PPSQ-10 (made by Shimadzu Seisakusho K.K.), to determine the N-terminal amino acid sequence of the enzyme. The result is as follows:

### Example 7

### Production of Cis-3-hydroxy-L-proline:

The enzyme reaction by use of purified L-proline-3-hydroxylase obtained in Example 5 was carried out. The reaction mixture was composed of 200 mM TES buffer (pH 7.0), 20 mM L-proline, 20 mM 2-ketoglutarate, 5 mM L-ascorbic acid, 1 mM ferrous sulfate, and 106 U of purified enzyme preparation, the total volume being 100 µl. The reaction was carried out at 35°C for 30 minutes. As a result of the reaction, 18 mM (2.4 g/l) of cis-3-hydroxy-L-proline was produced in the reaction mixture.

## Claims

1. A process for producing cis-3-hydroxy-L-proline, which comprises reacting a mixture of L-proline, 2-ketoglutaric acid and divalent iron ions with an enzyme source which catalyzes hydroxylation of L-proline at its 3-position in an aqueous medium, and recovering the cis-3-hydroxy-L-proline from the aqueous medium.

2. The process according to Claim 1, wherein the enzyme source is derived from a microorganism belonging to the genus Streptomyces or Bacillus.

3. The process according to Claim 2, wherein the enzyme source is selected from the group consisting of cells, a culture and processed cells, of the microorganism.

4. The process according to Claim 1 or 2, wherein the hydroxylation is carried out during cultivation of the microorganism.

5. The process according to Claims 2 or 3, wherein the enzyme source is an L-proline-3-hydroxylase obtainable from Streptomyces canus ATCC 12647, Streptomyces canus ATCC 12646, Streptomyces sp.TH1 (Deposit No.: FERM BP-4399), Bacillus sp.TH2 (Deposit No.: FERM BP-4397), Bacillus sp.TH3 (Deposit No.:FERM BP-4398) and, having the following physicochemical properties:
(1) Action and Substrate Specificity:
The enzyme catalyzes hydroxylation of L-proline at its 3-position in the presence of 2-ketoglutaric acid and divalent iron ions to produce cis-3-hydroxy-L-proline.
(2) Optimum pH Range:
The enzyme has an optimum pH range of 6.5 to 7.5 at 30°C for 20 minutes.
(3) Stable Range:
The enzyme is stable at pH values of 6.5 to 8.0, at 4°C for 23 hours.
(4) Optimum Temperature Range:
The optimum temperature range is 35 to 40°C at pH 7.0 for 15 minutes.
(5) Stable Temperature Range:
The enzyme is inactivated, at pH 7.5 and at 50°C for 30 minutes.
(6) Inhibitors:
The enzyme is inhibited by Zn⁺⁺, Cu⁺⁺ , Co⁺⁺ and Ba⁺⁺ metal ions and ethylenediaminetetraacetic acid (EDTA).
(7) Activation:
The enzyme does not need any cofactor for its activation. L-Ascorbic acid accelerates the activity of the enzyme.
(8) Km Value:
Km value is 0.49 mM for L-proline and is 0.11 mM for 2-ketoglutaric acid, when determined in a 100 mM N-tris(hydroxymethyl)methyl-2-aminoethanesulfonic acid (TES) buffer (pH 7.0) containing 5 mM L-ascorbic acid, 1 mM ferrous sulfate and a pre-determined amount of the enzyme.
(9) Isoelectric point:
The enzyme has an isoelectric point of 4.3 by Phastsystem™.
(10) Molecular Weight:
The enzyme has a molecular weight of 35,000 ± 5,000 daltons by sodium dodecylsulfate-polyacrylamide gel electrophoresis.
(11) N-terminal Amino Acid Sequence:
The enzyme has an N-terminal amino acid sequence illustrated by Sequence Number 1:

6. An L-proline-3-hydroxylase obtainable from Streptomyces canus ATCC 12647, Streptomyces canus ATCC 12646, Streptomyces sp.TH1 (Deposit No.: FERM BP-4399), Bacillus sp.TH2 (Deposit No.: FERM BP-4397), Bacillus sp.TH3 (Deposit No.: FERM BP-4398) and having the following physico-chemical properties:
(1) Action and Substrate Specificity:
The enzyme catalyzes hydroxylation of L-proline at its 3-position in the presence of 2-ketoglutaric acid and divalent iron ions to produce cis-3-hydroxy-L-proline.
(2) Optimum pH Range:
The enzyme has an optimum pH range of 6.5 to 7.5 at 30°C for 20 minutes.
(3) Stable pH Range:
The enzyme is stable at pH values of 6.5 to 8.0 at 4°C for 23 hours.
(4) Optimum Temperature Range:
The optimum temperature is in the range of 35 to 40°C at pH 7.0 for 15 minutes.
(5) Stable Temperature Range:
The enzyme is inactivated at pH 7.5 and at 50°C for 30 minutes.
(6) Inhibitors:
The enzyme is inhibited by Zn⁺⁺, Cu⁺⁺, Co⁺⁺ and Ba⁺⁺ metal ions and ethylenediaminetetraacetic acid (FDTA).
(7) Activation:
The enzyme does not need any cofactor for its activation. L-Ascorbic acid accelerates the activity of the enzyme.
(8) Km Value:
Km value is 0.49 mM for L-proline and is 0.11 mM for 2-ketoglutaric acid, when determined in a 100 mM N-tris(hydroxymethyl)methyl-2-aminoethanesulfonic acid (TES) buffer (pH 7.0) containing 5 mM L-ascorbic acid, 1 mM ferrous sulfate and a pre-determined amount of this enzyme.
(9) Isoelectric point:
The enzyme has an isoelectric point of 4.3 by Phastsystem™.
(10) Molecular Weight:
The enzyme has a molecular weight of 35,000 ± 5,000 daltons by sodium dodecylsulfate-polyacrylamide gel electrophoresis.
(11) N-terminal Amino Acid Sequence:
The enzyme has an N-terminal amino acid sequence illustrated by Sequence Number 1

7. The L-proline-3-hydroxylase according to Claim 6, which is derived from a microorganism belonging to the genus Streptomyces.

8. A process for producing L-proline-3-hydroxylase which catalyzes the hydroxylation of L-proline at its 3-position in the presence of 2-ketoglutaric acid and divalent iron ions comprising cultivating in a culture medium a microorganism belonging to the genus Streptomyces and having the ability to produce the L-proline-3-hydroxylase and recovering the L-proline-3-hydroxylase.

## Patentansprüche

1. Ein Verfahren zur Herstellung von cis-3 Hydroxy-L-prolin, umfassend die Umsetzung eines Gemisches von L-Prolin, 2-Ketoglutarsäure und zweiwertigen Eisenionen mit einer Enzymquelle, die die Hydroxylierung von L-Prolin in 3-Stellung in einem wäßrigen Medium katalysiert, und Gewinnung des cis-3-Hydroxy-L-prolins aus dem wäßrigen Medium.

2. Verfahren nach Anspruch 1, wobei die Enzymquelle abgeleitet ist von einem Mikroorganismus, der zu der Gattung der Streptomyces oder Bacillus gehört.

3. Verfahren nach Anspruch 2, wobei die Enzymquelle ausgewählt ist aus der Gruppe bestehend aus Zellen, einer Zellkultur und aufgearbeiteten Zellen des Mikroorganismus.

4. Verfahren nach Anspruch 1 oder 2, wobei die Hydroxylierung während der Kultivierung des Mikroorganismus ausgeführt wird.

5. Verfahren nach Anspruch 2 oder 3, wobei die Enzymquelle eine L-Prolin-3-hydroxylase ist, erhältlich aus Streptomyces canus ATCC 12647, Streptomyces canus ATCC 12646, Streptomyces sp.TH1 (Hinterlegungsnummer: FERM BP-4399), Bacillus sp.TH2 (Hinterlegungsnummer: FERM BP-4397), Bacillus sp.TH3 (Hinterlegungsnummer: FERM BP-4398) und mit den nachstehenden physikochemischen Eigenschaften:
(1) Wirkungs- und Substratspezifität:
Das Enzym katalysiert die Hydroxylierung von L-Prolin in seiner 3-Stellung in Gegenwart von 2-Ketoglutarsäure und zweiwertigen Eisenionen, um cis-3-Hydroxy-L-prolin zu bilden.
(2) Optimaler pH-Bereich:
Das Enzym hat einen optimalen pH-Bereich von 6,5 bis 7,5 bei 30°C für 20 Minuten.
(3) Stabiler pH-Bereich:
Das Enzym ist stabil bei pH-Werten von 6,5 bis 8,0 bei 4°C für 23 Stunden.
(4) Optimaler Temperaturbereich:
Der optimale Temperaturbereich liegt bei 35 bis 40°C bei einem pH-Wert von 7,0 für 15 Minuten.
(5) Stabiler Temperaturbereich:
Das Enzym wird inaktiviert bei einem pH-Wert von 7,5 und bei 50°C für 30 Minuten.
(6) Inhibitoren:
Das Enzym wird durch Zn⁺⁺, Cu⁺⁺, Co⁺⁺ und Ba⁺⁺ Metallionen und Ethylendiamintetraessigsäure (EDTA) inhibiert.
(7) Aktivierung:
Das Enzym benötigt keinen Cofaktor zu seiner Aktivierung. L-Ascorbinsäure erhöht die Aktivität des Enzyms.
(8) Km-Wert:
Der Km-Wert beträgt 0,49 mM für L-Prolin und 0,11 mM für 2-Ketoglutarsäure, wenn er in einem 100 mM N-Tris-(hydroxymethyl)-methyl-2-aminoethansulfonsäure-(TES)-Puffer (pH 7,0), enthaltend 5 mM L-Ascorbinsäure, 1 mM Eisen(II)-sulfat und eine vorbestimmte Menge des Enzyms, bestimmt wird.
(9) Isolelektrischer Punkt:
Das Enzym hat einen isoelektrischen Punkt von 4,3 nach Phastsystem™.
(10) Molekulargewicht:
Das Enzym hat ein Molekulargewicht von 35.000 ± 5.000 Dalton gemäß Natriumdodecylsulfat-Polyacrylamid-Gelelektrophorese.
(11) N-terminale Aminosäuresequenz:
Das Enzym hat eine N-terminale Aminosäuresequenz, wie sie unter der Sequenz-Nummer 1 dargestellt ist:

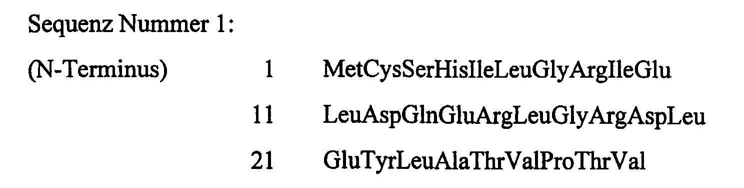

6. Eine L-Prolin-3-hydroxylase, erhältlich aus Streptomyces canus ATCC 12647, Streptomyces canus ATCC 12646, Streptomyces sp.TH1 (Hinterlegungsnummer: FERM BP-4399), Bacillus sp.TH2 (Hinterlegungsnummer: FERM BP-4397), Bacillus sp.TH3 (Hinterlegungsnummer: FERM BP-4398) und mit den nachstehenden physikochemischen Eigenschaften:
(1) Wirkungs- und Substratspezifität:
Das Enzym katalysiert die Hydroxylierung von L-Prolin in seiner 3-Stellung in Gegenwart von 2-Ketoglutarsäure und zweiwertigen Eisenionen, um cis-3-Hydroxy-L-prolin zu bilden.
(2) Optimaler pH-Bereich:
Das Enzym hat einen optimalen pH-Bereich von 6,5 bis 7,5 bei 30°C für 20 Minuten.
(3) Stabiler pH-Bereich:
Das Enzym ist stabil bei pH-Werten von 6,5 bis 8,0 bei 4°C für 23 Stunden.
(4) Optimaler Temperaturbereich:
Der optimale Temperaturbereich liegt bei 35 bis 40°C bei einem pH-Wert von 7,0 für 15 Minuten.
(5) Stabiler Temperaturbereich:
Das Enzym wird inaktiviert bei einem pH-Wert von 7,5 und bei 50°C für 30 Minuten.
(6) Inhibitoren:
Das Enzym wird durch Zn⁺⁺, Cu⁺⁺, Co⁺⁺ und Ba⁺⁺ Metallionen und Ethylendiamintetraessigsäure (EDTA) inhibiert.
(7) Aktivierung:
Das Enzym benötigt zu seiner Aktivierung keinen Cofaktor. L-Ascorbinsäure erhöht die Aktivität des Enzyms.
(8) Km-Wert:
Der Km-Wert beträgt 0,49 mM für L-Prolin und 0,11 mM für 2-Ketoglutarsäure, wenn er in einem 100 mM N-Tris-(hydroxymethyl)-methyl-2-aminoethansulfonsäure-(TES)-Puffer (pH 7,0), enthaltend 5 mM L-Ascorbinsäure, 1 mM Eisen(II)-sulfat und eine vorbestimmte Menge des Enzyms, bestimmt wird.
(9) Isolelektrischer Punkt:
Das Enzym hat einen isoelektrischen Punkt von 4,3 nach Phastsystem™.
(10) Molekulargewicht:
Das Enzym hat ein Molekulargewicht von 35.000 ± 5.000 Dalton gemäß Natriumdodecylsulfat-Polyacrylamid-Gelelektrophorese.
(11) N-terminale Aminosäuresequenz:
Das Enzym hat eine N-terminale Aminosäuresequenz, wie sie unter der Sequenz-Nummer 1 dargestellt ist:

7. L-Prolin-3-hydroxylase gemäß Anspruch 6, die abgeleitet ist von einem Mikroorganismus, der zu der Gattung der Streptomyces gehört.

8. Ein Verfahren zur Herstellung von L-Prolin-3-hydroxylase, die die Hydroxylierung von L-Prolin in seiner 3-Stellung in Gegenwart von 2-Ketoglutarsäure und zweiwertigen Eisenionen katalysiert, umfassend das Kultivieren in einem Kulturmedium eines Mikroorganismus, der zu der Gattung der Streptomyces gehört und die Fähigkeit besitzt, L-Prolin-3-hydroxylase zu bilden, und Gewinnung der L-Prolin-3-hydroxylase.

## Revendications

1. Procédé de préparation de la cis-3-hydroxy-L-proline, qui comporte le fait de faire réagir dans un milieu aqueux un mélange de L-proline, d'acide 2-cétoglutarique et d'ions de fer divalent, en présence d'une source d'enzyme catalysant l'hydroxylation en position 3 de la L-proline, et le fait de récupérer la cis-3-hydroxy-L-proline dans le milieu aqueux.

2. Procédé conforme à la revendication 1, dans lequel la source d'enzyme provient d'un microorganisme appartenant au genre Streptomyces ou Bacillus.

3. Procédé conforme à la revendication 2, dans lequel la source d'enzyme est choisie dans l'ensemble constitué par des cellules, une culture et des cellules traitées de microorganisme.

4. Procédé conforme à la revendication 1 ou 2, dans lequel l'hydroxylation est réalisée au cours de l'opération de culture du microorganisme.

5. Procédé conforme à la revendication 2 ou 3, dans lequel l'enzyme de la source est une L-proline 3-hydroxylase qu'on peut obtenir à partir de Streptomyces canus ATCC 12647, Streptomyces canus ATCC 12646, Streptomyces sp. TH1 (numéro de dépôt FERM BP-4399), Bacillus sp. TH2 (numéro de dépôt FERM BP-4397) ou Bacillus sp. TH3 (numéro de dépôt FERM BP-4398), et qui présente les propriétés physicochimiques suivantes :
1) Action et spécificité pour un substrat
Cette enzyme catalyse l'hydroxylation en position 3 de la L-proline, en présence d'acide 2-cétoglutarique et d'ions de fer divalent, ce qui donne de la cis-3-hydroxy-L-proline.
2) Domaine optimal de pH
Pour cette enzyme, le domaine optimal de pH va de 6,5 à 7,5, à 30 °C, pendant 20 minutes.
3) Intervalle de pH de stabilité
Cette enzyme reste stable pendant 23 heures, à 4 °C, à un pH valant de 6,5 à 8,0.
4) Domaine optimal de température
Le domaine optimal de température va de 35 à 40 °C, à pH 7,0, pendant 15 minutes.
5) Stabilité vis-à-vis de la température
Cette enzyme est inactivée quand on la laisse à 50 °C pendant 30 minutes, à pH 7,5.
6) Inhibiteurs
Cette enzyme est inhibée par les ions métalliques Zn²⁺, Cu²⁺, Co²⁺ et Ba²⁺, et par l'acide éthylènediamine-tétracétique (EDTA).
7) Activation
Pour être activée, cette enzyme n'a pas besoin d'un cofacteur, mais l'acide L-ascorbique favorise son activité.
8) Valeurs de Kₘ
Kₘ vaut 0,49 mM pour la L-proline et 0,11 mM pour l'acide 2-cétoglutarique, valeurs déterminées en solution tampon à 100 mM de TES (acide N-tris(hydroxyméthyl)méthyl-2-aminoéthanesulfonique) (pH 7,0) contenant 5 mM d'acide L-ascorbique, 1 mM de sulfate ferreux et une quantité connue d'enzyme.
9) Point isoélectrique
Le point isoélectrique de cette enzyme, déterminé au moyen d'un appareil Phastsystem®, vaut 4,3.
10) Masse moléculaire
La masse moléculaire de cette enzyme, déterminée par électrophorèse en gel de polyacrylamide en présence de dodécylsulfate de sodium, vaut 35 000 ± 5 000 daltons.
11) Séquence N-terminale d'acides aminés
La séquence N-terminale d'acides aminés de cette enzyme est présentée ci-dessous (Séquence n° 1) :

6. L-proline 3-hydroxylase qu'on peut obtenir à partir de Streptomyces canus ATCC 12647, Streptomyces canus ATCC 12646, Streptomyces sp. TH1 (numéro de dépôt FERM BP-4399), Bacillus sp. TH2 (numéro de dépôt FERM BP-4397) ou Bacillus sp. TH3 (numéro de dépôt FERM BP-4398), et qui présente les propriétés physicochimiques suivantes :
1) Action et spécificité pour un substrat
Cette enzyme catalyse l'hydroxylation en position 3 de la L-proline, en présence d'acide 2-cétoglutarique et d'ions de fer divalent, ce qui donne de la cis-3-hydroxy-L-proline.
2) Domaine optimal de pH
Pour cette enzyme, le domaine optimal de pH va de 6,5 à 7,5, à 30 °C, pendant 20 minutes.
3) Intervalle de pH de stabilité
Cette enzyme reste stable pendant 23 heures, à 4 °C, à un pH valant de 6,5 à 8,0.
4) Domaine optimal de température
Le domaine optimal de température va de 35 à 40 °C, à pH 7,0, pendant 15 minutes.
5) Stabilité vis-à-vis de la température
Cette enzyme est inactivée quand on la laisse à 50 °C pendant 30 minutes, à pH 7,5.
6) Inhibiteurs
Cette enzyme est inhibée par les ions métalliques Zn²⁺, Cu²⁺, Co²⁺ et Ba²⁺, et par l'acide éthylènediamine-tétracétique (EDTA).
7) Activation
Pour être activée, cette enzyme n'a pas besoin d'un cofacteur, mais l'acide L-ascorbique favorise son activité.
8) Valeurs de Kₘ
Kₘ vaut 0,49 mM pour la L-proline et 0,11 mM pour l'acide 2-cétoglutarique, valeurs déterminées en solution tampon à 100 mM de TES (acide N-tris(hydroxyméthyl)méthyl-2-aminoéthanesulfonique) (pH 7,0) contenant 5 mM d'acide L-ascorbique, 1 mM de sulfate ferreux et une quantité connue d'enzyme.
9) Point isoélectrique
Le point isoélectrique de cette enzyme, déterminé au moyen d'un appareil Phastsystem®, vaut 4,3.
10) Masse moléculaire
La masse moléculaire de cette enzyme, déterminée par électrophorèse en gel de polyacrylamide en présence de dodécylsulfate de sodium, vaut 35 000 ± 5 000 daltons.
11) Séquence N-terminale d'acides aminés
La séquence N-terminale d'acides aminés de cette enzyme est présentée ci-dessous (Séquence n° 1) :

7. L-proline 3-hydroxylase conforme à la revendication 6, qui provient d'un microorganisme appartenant au genre Streptomyces.

8. Procédé de production d'une L-proline 3-hydroxylase qui catalyse l'hydroxylation en position 3 de la L-proline, en présence d'acide 2-cétoglutarique et d'ions de fer divalent, lequel procédé comporte le fait de cultiver, dans un milieu de culture, un microorganisme appartenant au genre Streptomyces et capable de produire une L-proline 3-hydroxylase, et le fait de récupérer la L-proline 3-hydroxylase.
